# EUROPEAN PATENT APPLICATION

(11) **EP 1 475 381 A1**
(43) Date of publication of application: **10.11.2004**
(21) Application number: 02701303.6
(22) Date of filing: 14.02.2002
(51) Int. Cl.: C07D 487/18, A61K 7/38, A61K 33/06

(54) **ALUMINIUM AND HEXAMETHYLENETETRAMINE COMPLEX AND THE APPLICATIONS THEREOF**

(71) Applicant: Quimversion, S.L., 50008 Zaragoza (ES)
(72) Inventor: TANCO SALAS, Jesus-Maria, E-50500 Tarazona (ES)
(74) Representative: PROPI, S.L.
(86) International application number: PCT/ES2002/000070
(87) International publication number: WO 2003/068777

(57) **Abstract**

The aluminum and hexamethylenetetramine (Al-HMT) complex is obtained by mixing a source of aluminum and HMT and optionally leaving the resulting mixture to sit. The Al-HMT complex has deodorant and therapeutic properties, and it is useful for elaborating cosmetic and pharmaceutical compositions. Furthermore, said Al-HMT complex has the capacity to trap substances which can subsequently be released, it therefore being useful as a carrier of said substances.

## Description

### BACKGROUND OF THE INVENTION

The invention refers to an aluminum and hexamethylenetetramine complex and to the use thereof in the elaboration of cosmetic or pharmaceutical compositions, as well as a carrier of substances of interest.

### BACKGROUND OF THE INVENTION

Among those factors responsible for bad body order is the excessive secretion of sweat due to which the development of microorganisms is favored which, when the substance secreted by sweating degrade, cause bad odor.

To fight the bad order, deodorant compositions are used which can be presented in a multitude of forms, for example, in solid, semi-solid, liquid or aerosol composition forms.

Numerous compounds are known which are capable of exercising a deodorant effect. Among said compounds is hexamethylenetetramine (HMT), a compound exercising an inhibiting effect on the bacterial flora of the skin, thus being able to remove or reduce one of the causes responsible for bad body odor.

The use of HMT in products intended for fighting bad body odor has been disclosed in several publications, for example, in British patent GB 1,525,971, which discloses a deodorant for bodily use comprising HMT, zinc oxide, starch, vaseline and perfume, and in Spanish patent application number P9800863, which discloses an emulsion form composition comprising an oily phase comprising between 2% and 6% by weight of zinc oxide with regard to the total weight of the composition, and an aqueous phase comprising between 2% and 6% by weight of hexamethylenetetramine with regard to the total weight of the composition, hexamethylenetetramine being useful for elaborating cosmetic products particularly intended for fighting bad odor of the sole of the foot.

Although numerous products are known for the treatment of bad body odor, there is still the need to develop new products for the purpose of increasing the arsenal of remedies to fight excessive sweating and the bad odor associated with it. This invention provides a solution to said existing need.

### DETAILED DESCRIPTION OF THE INVENTION

The invention provides an aluminum and hexamethylenetetramine (HMT) complex, hereinafter, Al-HMT complex, useful as an antitranspirant, deodorant, antibacterial and/or disinfectant, therefore it is suitable for elaborating cosmetic and pharmaceutical compositions. Furthermore, said Al-HMT complex has the capacity to "trap" or retain substances which can subsequently be released, it is therefore useful as a carrier of said substances.

The Al-HMT complex is a solid product forming a type of three-dimensional network in which different substances can be trapped. These substances which can be trapped in the Al-HMT complex can be either substances completing or reinforcing the deodorant action of said Al-HMT complex or therapeutically active substances which completing or reinforcing the therapeutic action of said Al-HMT complex. Alternatively, said substances which can be trapped in the Al-HMT complex can be substances which exercise an effect different from that of the Al-HMT complex, for example, substances useful as air fresheners, odor neutralizers, insecticides, insect repellants, etc. In this case, the Al-HMT complex acts as a carrier of said substances.

The Al-HMT complex can be obtained by means of a process comprising mixing its components (Al and HMT) or a source thereof, optionally allowing the resulting mixture to sit.

Any compound capable of providing aluminum in a sufficient amount so that it reacts with HMT and forms the Al-HMT complex can be used as a source of aluminum. For example, said source of aluminum can be aluminum chlorhydroxide [Al₂(OH)₅Cl·(xH₂O)], aluminum trichloride, aluminum acetate, aluminum and potassium sulfate, etc. In a particular embodiment, said source of aluminum is aluminum chlorhydroxide, optionally of pharmaceutical quality.

The source of HMT is, normally, HMT itself.

The Al-HMT complex precursors (i.e. the source of aluminum and HMT) are water soluble compounds. Therefore, said precursors can be found in aqueous solution form. Alternatively, they can also be found in aqueous alcohol solution form, in which said alcohol can be a monoalcohol, a diol or a polyol, for example, ethanol, propylene glycol, glycerol, etc.

Obtaining said Al-HMT complex is carried out by means of a process comprising mixing the components or the precursors thereof in an aqueous or aqueous alcohol medium. The formation of the Al-HMT complex can be practically immediate after the mixture of the components or the precursors thereof, or, alternatively, after letting the resulting mixture sit until the Al-HMT complex is formed, which is clearly evident due to the appearance of a solid.

The pH of the reaction medium must generally be equal to or greater than 2.5, since the Al-HMT complex is unstable and is destroyed by decomposition of the HMT at pH values lower than 2.5. In the case that the pH of the medium were lower than 2.5, a pH corrector, such as a base, would be added.

The ratio between the components or precursors thereof (source of aluminum and HMT) can range within a broad interval, practically in any weight ratio. In fact, it has been observed that the formation of the Al-HMT complex always occurs when the starting materials are aluminum chlorhydroxide and HMT, regardless of the ratios and the relative concentrations of each one of said compounds, only varying the necessary sitting time, after the mixture of said components, according to the concentration of said components and the polarity of the solvent. In a particular embodiment, the Al:HMT weight ratio present in the Al-HMT complex is comprised between 1:0.04 and 1:6.

The process for obtaining the Al-HMT complex can be carried out within a broad temperature interval. In a particular embodiment, said process is carried out at a temperature comprised between 13°C and 37°C. Different tests have shown (see Example 1) that (i) at the same temperature, the Al-HMT complex is obtained more quickly the larger the concentrations of Al and HMT are; and that (ii) at equal concentrations, said Al-HMT complex is obtained more quickly the higher the temperature is.

Due to the properties of the Al-HMT complex, the latter has multiple applications, cosmetic applications due to its deodorant and antitranspirant effect, as well as pharmaceutical applications due to its disinfectant, bactericidal or bacteriostatic (antiseptic) effect. Furthermore, due to its capacity to trap substances which can subsequently be released, it is useful as a carrier of said substances.

Therefore, in another aspect, the invention provides a cosmetic composition, hereinafter cosmetic composition of the invention, comprising said Al-HMT complex, or the precursors (i.e. the source of aluminum and HMT) thereof forming said complex during the application of said composition, together with, optionally, one or more cosmetically active substances.

In the sense used in this description, the expression "cosmetically active substance" includes any substance exercising a cosmetic effect, for example, essences, perfumes, moisturizers, deodorants, urea, hyaluronidase, extracts, for example hamamelis extract, hydroglycolic extract of seaweed or of ivy, etc. Said cosmetically active substances are known by persons skilled in the art.

Although the Al-HMT complex has an antitranspirant and deodorant effect *per se,* probably due, although it is not the intention to be bound by any theory, to the inhibiting effect on the bacterial flora of the skin of the HMT present in the Al-HMT complex, and to the inhibiting effect on the sweat gland of the aluminum also present in the Al-HMT complex, said deodorant and antitranspirant effect of the Al-HMT complex can be complemented or enhanced by the action of other substances which can be trapped in the Al-HMT complex. Effectively, as previously mentioned, due to the structure of the Al-HMT complex, the latter has the capacity to trap substances which can complement or enhance its own deodorant effect with the effects of another or other cosmetically active substance(s) which can be trapped by said complex, for example, perfumes complementing its deodorant action, moisturizers compensating the drying effect, additional deodorants and/or antitranspirants enhancing or reinforcing the deodorant and antitranspirant effect of the Al-HMT complex itself, etc.

The Al-HMT complex can be present as such in the cosmetic composition of the invention or, alternatively, said Al-HMT complex can be formed *in situ,* i.e. on the skin of the person or animal to whom a cosmetic composition is applied which comprises the Al-HMT complex precursors.

In the event that the cosmetic composition of the invention contains one or more cosmetically active substances in addition to the Al-HMT complex, said cosmetic composition can be obtained by means of a process comprising the mixture of the Al-HMT complex, or the precursors (source of aluminum and HMT) thereof, with said cosmetically active substance or substances.

The invention therefore provides a cosmetic product comprising said cosmetic composition of the invention together with one or more cosmetically acceptable additives and/or carriers.

The cosmetic product provided by this invention can be presented in different presentation forms. Generally, said presentation forms can be solid, liquid or semi-solid forms, for example sticks, ointments, creams, milks, emulsions, etc., therefore said cosmetic product provided by the invention will include the carriers and additives cosmetically acceptable for the chosen presentation form. A review of the different presentation forms of cosmetic products and of the carriers and additives suitable for said presentation forms can be found in the book entitled "Cosmetología Teórica-Práctica" edited by the Consejo General de Colegios Oficiales de Farmacéuticos, 30th Edition (1985). In a particular embodiment, the cosmetic product provided by this invention is presented in emulsion form, further comprising other hydrosoluble as well as liposoluble substances of interest. In another particular embodiment, the cosmetic product provided by this invention is presented in the form of more or less thick "milks" or emulsions, frequently used in roll-ons, a widely used formulation in deodorant cosmetics.

The cosmetic product provided by this invention can be obtained by means of a process comprising mixing the Al-HMT complex, or the precursors (source of aluminum and HMT) thereof, with one or more cosmetically acceptably additives and/or carriers, and, optionally, with one or more cosmetically active substances.

In another aspect, the invention provides a pharmaceutical composition, hereinafter pharmaceutical composition of the invention, comprising said Al-HMT complex, or the precursors (source of aluminum and HMT) thereof, forming said complex during the application of said composition, together with, optionally, one or more pharmaceutically active substances and/or one or more pharmaceutically acceptable excipients.

In the sense used in this description, the expression "pharmaceutically active substance" includes any substance exercising a therapeutic effect, for example urea (a substance which can exercise a therapeutic as well as cosmetic effect), antiseptics, antibiotics, alkaloids, hydrosoluble vitamins, hydrosoluble calcium or magnesium salts, etc.

Although the Al-HMT complex exercises a therapeutic effect *per se,* probably due, although it is not the intention to be bound by any theory, to the antibacterial and disinfectant effect of the HMT (The Merck Index, 12th Edition, 1996, item 6036) present in the Al-HMT complex, said therapeutic effect of the Al-HMT complex can be complemented or enhanced by the action of other substances which can be trapped in the Al-HMT complex. Effectively, as previously mentioned, due to the structure of the Al-HMT complex, the latter has the capacity to trap substances which can complement or enhance its own therapeutic effect with the effects of another or other pharmaceutically active substance(s) which can be trapped by said complex, for example, urea, antiseptics, antibiotics, alkaloids, hydrosoluble vitamins, hydrosoluble calcium or magnesium salts, etc., complementing or reinforcing its therapeutic effect.

Generally, the pharmaceutical composition of the invention can be presented in a solid, liquid or semi-solid dosage form. In a particular embodiment, said dosage form is suitable for the topical application thereof, for which said composition includes the necessary pharmaceutically acceptable excipients. Other dosage forms of the pharmaceutical composition of the invention include solid oral dosage forms, which include the suitable excipients. A review of the different presentation forms of active substances and of the excipients necessary for its elaboration can be found in the book entitled "Tratado de Farmacia Galénica", C. Fauli i Trillo, Luzán 5, S.A. de Ediciones, 10th Edition, 1993.

The pharmaceutical composition of the invention can be obtained by means of a process comprising mixing the Al-HMT complex, or the precursors (source of aluminum and HMT) thereof, with one or more pharmaceutically acceptable excipients and, optionally, with one or more pharmaceutically active substances.

The applications disclosed heretofore refer to the use of the Al-HMT complex as an active substance *per se,* i.e. as a substance capable of exercising a cosmetic or therapeutic effect alone in the human or animal body. However, said Al-HMT complex can also be used as a carrier of substances of interest due to its capacity to trap said substances which can subsequently be released. In this case, the Al-HMT complex is not used because of its own activity (cosmetic and/or pharmaceutical), but rather solely and exclusively as a carrier of said substances of interest.

In the sense used in this description, the expression "substance of interest" includes any volatile or nonvolatile substance exercising a desired effect, such as a cosmetic or therapeutic effect, on the human or animal body, or a desired effect on the environment, for example, an air freshener, odor neutralizer, insecticide, insect repellant, disinfectant, etc. Non-limiting illustrative examples of substances of interest exercising a cosmetic or therapeutic effect on the human or animal body, which can be carried by the carrier of the invention, include magnesium lactate, vitamin C, essences with therapeutic activities (cedar, nutmeg, turpentine, menthol, eucalyptus, thymol, etc.), permethrin, azelaic acid, 1-piperidinecarboxylic acid, etc. Non-limiting illustrative examples of substances of interest not exercising a cosmetic or therapeutic effect on the human or animal body, but rather a desired effect on the environment, which can be carried by the carrier of the invention, include lemon oil, perfumes and aromas, para-dichlorobenzene, camphor, pyrethroids, etc.

Therefore, in another aspect, the invention provides a carrier of substances of interest, hereinafter carrier of the invention, whose composition comprises the Al-HMT complex and, optionally, one or more additives.

In a particular embodiment, the carrier of the invention comprises between 0.6% and 90% by weight with regard to the total weight of said Al-HMT complex.

The additives which can optionally be present in the carrier of the invention include the solvents in which the Al-HMT complex precursors, pH correctors and the substances favoring the formation and/or stability of the Al-HMT complex are dissolved.

The solvents in which the Al-HMT complex precursors (source of aluminum and HMT) are dissolved include water or water and alcohols, which can be trapped in the formed Al-HMT complex. As previously mentioned, the source of aluminum as well as HMT are hydrosoluble, therefore, generally, the water is normally the solvent of choice for obtaining the Al-HMT complex; however, due to the fact that among the substances of interest, there can be substances that are insoluble or barely soluble in water, although they are soluble in alcohol, for example, essences, the carrier of the invention can contain, in addition to water, said alcohol, optionally trapped by the Al-HMT complex.

The pH correctors are substances which are optionally added to prevent the pH of the medium in which the Al-HMT complex is in from being lower than 2.5, since the Al-HMT complex is unstable and is destroyed by decomposition of the HMT at pH values lower than 2.5. On the contrary, at equal or higher pH values, preferably equal to or higher than 4.5, the Al-HMT complex is stable. The use of a pH corrector depends, among others, on the precursors used. Thus, when stoichiometric amounts of aluminum chlorhydroxide and HMT react, the pH obtained is between 5.2 and 5.7, therefore the Al-HMT complex obtained is stable in the reaction medium and does not require the addition of a pH corrector; however, when the source of aluminum is aluminum trichloride, due to the acid character of its aqueous solution, it is convenient to add a base in order to increase the pH of the reaction medium and to be able to form the Al-HMT complex.

The substances favoring the formation and/or stability of the Al-HMT complex include substances which increase the formation rate of the Al-HMT complex and/or its stability, for example, the addition of alcohol or of small amounts of zinc acetate.

Therefore, the invention provides a product comprising one or more substances of interest together with a carrier of the invention. Said substance of interest includes any volatile or nonvolatile substance exercising a desired effect, such as a cosmetic or therapeutic effect, on the human or animal body, or a desired effect on the environment, for example an air freshener, odor neutralizer, insecticide, insect repellant, disinfectant, etc.

In a particular embodiment, said substance of interest is a substance exercising an air freshener effect, for example lemon essence. In this case, the invention provides an air freshener comprising the carrier of the invention together with one or more substances with an air freshener effect. The carrier of the invention can be present in an amount comprised between 1% and 99% by weight with regard to the total weight of the air freshener. Said air freshener can be obtained by mixing the Al-HMT complex precursors with the substance or substances with an air freshener effect and by optionally adding one or more additives, selected among alcohols, pH correctors and substances favoring the formation and stability of the Al-HMT complex.

To obtain an air freshener with a small amount of Al-HMT complex and a large amount of volatile substances, the Al-HMT complex precursors are mixed with the substance or substances with air freshener effect and one or more additives are added, selected among pH correctors (where applicable) and substances favoring the formation and stability of the Al-HMT complex, for example an alcohol and zinc acetate.

Alternatively, an air freshener can be obtained with a high concentration of Al-HMT complex, for example 45% by weight with regard to the total weight, mixing the Al-HMT complex precursors in aqueous alcohol solutions with a high concentration in precursors together with the substance or substances with air freshener effect and, subsequently, removing all or a majority of the solvent or solvents from the solutions of the precursors by conventional methods, for example by means of using a hygroscopic substance.

In another particular embodiment, the substance of interest is a substance with an insecticide effect for either the environment or human or animal use. In the case of environment insecticides, these are normally presented in solid form containing the environment insecticide or insecticides carried in the carrier of the invention. In the case of insecticides for human or animal use, said insecticides can be presented in the form of a stick, emulsions, suspensions or creams, i.e. similar to how the cosmetic compositions of the invention are presented.

The following examples serve to illustrate the invention and should not be considered limiting of the scope thereof.

### EXAMPLE 1

### Obtaining the Al-HMT complex

Different tests related to obtaining the Al-HMT complex have been carried out using different media, and the ratio between temperature and concentration has been studied.
- Test A:: For a final 18% Al and 18% HMT concentration in the Al-HMT complex, using distilled water as a solvent and carrying out the mixture at different temperatures, the Al-HMT complex was obtained (solidification) in the following times:

| Temperature | Time |
|---|---|
| 37°C | 1 day |
| 21°C | 19 days |
| 7°C | no solidification was observed after 1 month |

- Test B:: For a final 10% Al and 10% HMT concentration in the Al-HMT complex, using distilled water as a solvent and carrying out the mixture at different temperatures, the Al-HMT complex was obtained (solidification) in the following times:

| Temperature | Time |
|---|---|
| 37°C | 4 days |
| 21°C | 1 month |
| 7°C | no solidification was observed after 1 month |

- Test C:: For a final 5% Al and 5% HMT concentration in the Al-HMT complex, using distilled water as a solvent and carrying out the mixture at different temperatures, the Al-HMT complex was obtained (solidification) in the following times:

| Temperature | Time |
|---|---|
| 37°C | 27 days |
| 21°C | no solidification was observed after 1 month |
| 7°C | no solidification was observed after 1 month |

- Test D:: For a final 18% Al and 18% HMT concentration in the Al-HMT complex, using an aqueous alcohol solution as a solvent, yielding a 60° final aqueous alcohol solvent, and at a temperature of 21°C, the solidification is instantaneous. With smaller alcohol gradations, for example 35°, the solidification was obtained by formation of the Al-HMT complex in a few minutes.

These tests clearly show that at equal temperature, the Al-HMT complex is obtained more quickly the higher the Al and HMT concentrations are; and that at equal concentrations, said Al-HMT complex is obtained more quickly the higher the temperature is.

### EXAMPLE 2

### Air freshener

This test illustrates the possibility of using the Al-HMT complex as a carrier of volatile substances (lemon essence). To do this, the following solutions were separately prepared:
- Solution A:: 2 g of lemon essence in 33 g of absolute ethanol;
- Solution B:: 15 g of aluminum chlorhydroxide in 15 g of water; and
- Solution C:: 15 g of HMT in 20 mg of water.

Solutions A and B were mixed and solution C was subsequently added. The resulting mixture was left to sit until its solidification at room temperature.

### EXAMPLE 3

### Pharmaceutical form comprising the Al-HMT complex and magnesium lactate

This test illustrates the possibility of using the Al-HMT complex as a carrier of nonvolatile substances. To do this, the following solutions were separately prepared:
- Solution A:: 10 g of magnesium lactate and 15 g of aluminum chlorhydroxide in 35 g of water; and
- Solution B:: 15 g of HMT in 25 g of water.

Solutions A and B were mixed and the resulting mixture was left to sit until its solidification, leaving it to dry at room temperature so that it would lose all or part of the water contained in the Al-HMT complex network, thereby obtaining a solid with a crystalline appearance, containing approximately 20% by weight of magnesium lactate, which can be administered in the form of powder (capsules), granules, etc., after studying the oral route toxicity of the Al-HMT complex.

### EXAMPLE 4

### Pharmaceutical form comprising the Al-HMT complex and vitamin C

This test illustrates the possibility of using the Al-HMT complex as a carrier of nonvolatile substances. To do this, the following solutions were separately prepared:
- Solution A:: 15 g of aluminum chlorhydroxide and 10 g of vitamin C in 35 g of water; and
- Solution B:: 15 g of HMT in 25 g of water.

Solutions A and B were mixed and the resulting mixture was left to sit until its solidification, leaving it to dry at room temperature so that it would lose all or part of the water contained in the Al-HMT complex network, thereby obtaining a solid with a crystalline appearance, containing approximately 20% by weight of vitamin C, which can be administered in the form of powder (capsules), granules, etc., after studying the oral route toxicity of the Al-HMT complex.

### EXAMPLE 5

### Insecticide comprising an Al-HMT complex as a carrier

4 g of a self-emulsifiable 25% permethrin oily solution were suspended in 36 g of water (Emulsion A), and it emulsified at 40°C. On the other hand, a solution of 15 g of aluminum chlorhydroxide in 15 g of water (Solution B) and another solution of 15 g of HMT in 15 g of water (Solution C) were prepared, the temperature of the water in both cases being 40°C. Then, Emulsion A was mixed with Solutions B and C, and the resulting mixture was left to sit, thereby obtaining a solid product. Furthermore, in this case, the Al-HMT complex was the external part of a solid O/W emulsion.

The insecticide can be applied topically, rubbing the area of the body to be treated with said solid product, for example the scalp (in cases of pediculosis) or the pubic area (in case of crabs).

### EXAMPLE 6

### Insect repellant

The following solutions were prepared separately:
- Solution A:: 20 g of 1-piperidinecarboxylic acid in 32 g of 96° ethanol;
- Solution B:: 8 g of aluminum chlorhydroxide in 16 g of water; and
- Solution C:: 8 g of HMT in 16 g of water.

Solutions A, B and C were mixed, and the resulting mixture was left to sit at room temperature, thereby obtaining a solid product.

The topical application of the insect repellant obtained is carried out by rubbing the skin with said solid product.

### EXAMPLE 7

### Acne treatment cream

The following solutions were prepared separately:
- Solution A:: 10 g of azelaic acid in 10 g of 96° ethanol;
- Solution B:: 10 g of aluminum chlorhydroxide in 17 g of water; and
- Solution C:: 10 g of HMT in 18 g of water.

Solutions A and B were mixed to homogeneity and heated at 70°C. Then, solution C, previously heated at 70°C, was added. The resulting mixture of solutions A, B and C was emulsified, slowly adding and under stirring 25 g of a W/O absorption base previously heated at 70°C, (Base D) consisting of mineral oil, lanolin alcohol, glyceryl oleate, ozokerite and petrolatum. It was subsequently left to cool to 40°C, maintaining stirring.

Azelaic acid will, for the most part, be enclosed in the three-dimensional Al-HMT complex network formed which, in turn, will be within the aqueous micelle of the emulsion and, when applied on the skin, the effect of the azelaic acid on acne is prolonged over time while the Al-HMT complex network retaining it decomposes.

### EXAMPLE 8

### Antibiotic cream

A cream containing erythromycin was prepared from the following solutions (separately):
- Solution A:: 2 g of erythromycin in 10 g of 96° ethanol;
- Solution B:: 10 g of aluminum chlorhydroxide in 20 g of water;
- Solution C:: 10 g of HMT in 23 g of water; and
- Base D:: 25 g of the absorption base from Example 7.

The cream was obtained following the process described in Example 7.

## Claims

1. An aluminum and hexamethylenetetramine complex.

2. A process for obtaining an aluminum and hexamethylenetetramine (Al-HMT) complex comprising mixing aluminum or a source of aluminum and hexamethylenetetramine (HMT) and, optionally leaving the resulting mixture to sit.

3. A process according to claim 1, wherein said source of aluminum is a compound capable of providing aluminum in a sufficient amount so that it reacts with the HMT and forms the Al-HMT complex.

4. A process according to claim 3, wherein said source of aluminum is selected among aluminum chlorhydroxide [Al₂(OH)₅Cl·(xH₂O)], aluminum trichloride, aluminum acetate, aluminum and potassium sulfate, and mixtures thereof.

5. A cosmetic composition comprising said Al-HMT complex according to claim 1, or the precursors thereof, a source of aluminum and HMT, which form said complex during the application of said cosmetic composition, together with, optionally, one or more cosmetically active substances.

6. A cosmetic composition according to claim 5, wherein said cosmetically active substance is selected among essences, perfumes, moisturizers, deodorants, urea, hyaluronidase, plant and/or seaweed extracts, and the mixtures thereof.

7. A cosmetic product comprising a cosmetic composition according to any of claims 5 or 6, together with one or more cosmetically acceptable additives and/or carriers.

8. A pharmaceutical composition comprising said Al-HMT complex according to claim 1, or the precursors thereof, a source of aluminum and HMT, which form said complex during the application of said pharmaceutical composition, together with, optionally, one or more pharmaceutically active substances and/or one or more pharmaceutically acceptable excipients.

9. A pharmaceutical composition according to claim 8, wherein said pharmaceutically active substance is selected among urea, antiseptics, antibiotics, alkaloids, hydrosoluble vitamins, hydrosoluble calcium or magnesium salts, and the mixtures thereof.

10. A carrier of substances of interest, whose composition comprises said Al-HMT complex according to claim 1 and, optionally, one or more additives.

11. A carrier according to claim 10, wherein said Al-HMT complex is present at a concentration comprised between 0.6% and 90% by weight with regard to the total weight of said carrier.

12. A carrier according to claim 10, wherein said substance of interest comprises a volatile or nonvolatile substance exercising a desired effect.

13. A carrier according to claim 12, wherein said substance of interest is selected among air fresheners, odor neutralizers, insecticides, insect repellants, disinfectants and the mixtures thereof.

14. A carrier according to claim 10, wherein said additives comprise solvents in which the Al-HMT complex precursors, pH correctors and/or substances favoring the formation and/or stability of the Al-HMT complex are dissolved in.

15. A carrier according to claim 14, wherein said solvents in which the Al-HMT complex precursors are dissolved in comprise water, alcohols and the mixtures thereof.

16. A carrier according to claim 14, wherein said additives comprise substances favoring the formation and/or stability of the Al-HMT complex selected among alcohols, zinc acetate and the mixtures thereof.

17. A product comprising one or more substances of interest together with a carrier according to any of claims 10 to 16.

18. A product according to claim 17, wherein said substance of interest comprises an air freshener, an odor neutralizer, an insecticide, an insect repellant or a disinfectant.

19. A product according to claim 17, selected among an air freshener, an insecticide, a repellant and a disinfectant.
